# EUROPEAN PATENT APPLICATION

(11) **EP 1 884 212 A1**
(43) Date of publication of application: **06.02.2008**
(21) Application number: 07252507.4
(22) Date of filing: 20.06.2007
(51) Int. Cl.: A61B 17/70

(54) **Spinal rod connector**

(30) Priority: 04.08.2006 US 462464
(71) Applicant: Zimmer Spine, Inc., Minneapolis, MN 55439-2027 (US)
(72) Inventor: Vaidya, Shailendra, Bloomington, MN 55420 (US); Tyagi, Kuldeep, Bloomington, MN 55420 (US); Hillyard, Angela L., Greenfield, MN 55373 (US)
(74) Representative: Findlay, Alice Rosemary

(57) **Abstract**

A system and method for fixing the relative position of vertebrae in a human spinal column utilizes a unique connector (11), vertebral anchors (14) and a longitudinal pre-shaped rod. The connector assemblies each have a pass-through aperture (12) for the rod. Each of the apertures is shaped such that at least 3 surfaces of contact are created between a variety of rod sizes and each of the connectors, thereby allowing secure and stable holding of the rod with respect to the other system components and the vertebrae.

## Description

This invention relates to an apparatus and method for fixing the relative location of human vertebrae in a spinal column, and more specifically a spinal fixation construct utilizing vertebrae-engaging members, spine rods and connectors between the rod and engaging members.

Surgically implanted spinal fixation systems are well known and are used to correct a variety of back structure problems, including those resulting from trauma as well as defective growth-related development of the spinal column. Apparatus to fix the relative location of human vertebrae are known and may consist of screws that are inserted into the vertebrae and are interconnected to a pre-shaped support rod by the use of rigid connectors or clamps.

Some spinal fixation systems contain features that allow securing the support rod to the rigid connectors or clamps. The rigid connectors and clamps, however, can only accommodate support rods of either one specific diameter or a very small range of diameters. In apparatus that can accommodate a range of rod diameters, the rod and connector interface is typically one made up of contact provided by a fastener or set-screw and a continuous curved surface of the connector. In other cases, as in systems where the connectors are clamps, a good match between the connector rod opening and the surface of the rod is difficult to achieve. In either situation, the connector does not adequately and securely hold the rod. In the case of a point-and-continuous-curved surface contact, rotation of the rod with respect to the connector is undesirably possible. In the case of a mismatched surface contact, rotation and lateral movement of the rod with respect to the connector is likewise possible. The result of these inadequate rod support systems is the potential for undesirable movement and misalignment of the spinal fixation system components.

Similarly, known connectors and clamps work, as indicated, with rods of diameters that are precisely compatible only with one specific type of connector or clamp, thereby limiting the options available to the surgeon who, in the midst of surgery, may be faced with a need to change the type of support rod to be used. This exact compatibility of connectors and rods requires a greater inventory of connectors to ensure different options are available to the surgeon in a timely fashion.

An apparatus that allows for a greater versatility and adaptability to rods of different diameters, so as to increase the options available to the surgeon in the midst of an operation would therefore be desirable. An apparatus that reduces the required size and type of inventory of spinal fixation systems or components that a surgeon must have on hand would similarly be desirable. Finally, a system that ensures secure contact between the rod and connectors, so as to firmly secure these components to each other, thereby ensuring the required rigidity and stability of the system once installed, is also desirable, especially in light of the dynamic environment to which such system will be exposed.

This invention provides a new and improved system and method for fixation of vertebrae in a human spinal column. The system in one embodiment includes two or more anchors adapted for coupling to the spinal column, a support rod adapted to hold the relative position of vertebrae in the column, connector assemblies interconnecting the rod and the anchors, and rod pass-through apertures in the connectors providing at least 3 surfaces of contact between the rod and each of the connectors.

One embodiment of the invention includes a polyaxial pedicle screw as the anchor with a threaded portion adapted for insertion into the spine, coupled to the connector having a pass-through polygonal aperture to receive and firmly secure the rod with a set screw. The set screw provides a first point of contact and two non-contiguous surfaces in the aperture provide two more independent cross-sectional points of contact.

This invention, at least in the preferred embodiments, allows stable and secure locking of the rod against the connector and ultimately against each of the vertebrae held by the system. Furthermore, this invention offers this advantage for rods in a range of diameters, thereby allowing a reduced inventory of spinal fixation system components.

The invention will now be further described by way of example with reference to the accompanying drawings in which:

FIG. 1 is a perspective view of one embodiment of the invention, showing, among others, the range of motion of a polyaxial pedicle screw and a connector assembly mounted to the screw;

FIG. 2 is a side view of the connector assembly of the embodiment of FIG. 1, along with a set screw to be received by the connector assembly;

FIG. 3 is a partial cross-sectional view of the connector assembly of FIGS. 1 and 2, showing a rod secured to the connector assembly;

FIG. 4 shows the same connector assembly of FIG. 3 securing a rod of a diameter larger than that shown on FIG. 3; and

FIGS. 5 and 6 are various views showing the shape of an aperture in the connector assembly according to one embodiment of this invention.

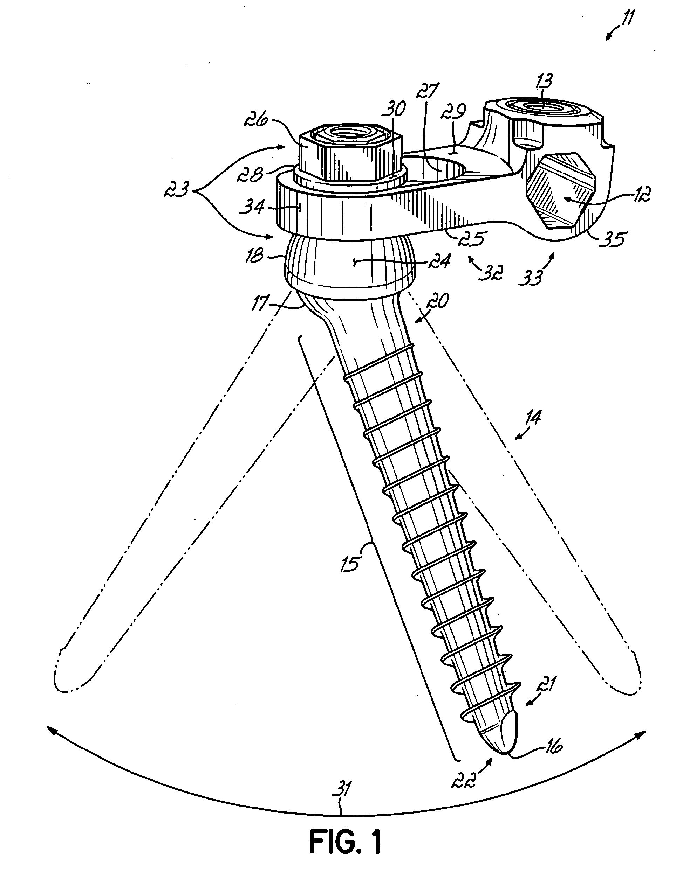

FIGS. 1-4 show a system for fixation of human spinal column vertebrae through the use of a pre-shaped rod 10 following a desired orientation of the column, a series of connectors 11 each having an aperture 12 allowing the rod 10 to pass through them, fasteners such as set screws 13 securing each of the connectors 11 to the rod 10, and vertebrae-engaging anchors such as pedicle screws 14 securing each of the connectors 11 to a vertebra. This system, therefore, along with the associated method of spinal column fixation, achieves the goal of defining and preserving a desired relative spatial orientation of the vertebrae. While this system is shown and described herein as a spinal fixation construct, it is readily applicable for use in other areas of the body such as the femur, tibia, fibula, humerus, ulna, radius, clavicle and others.

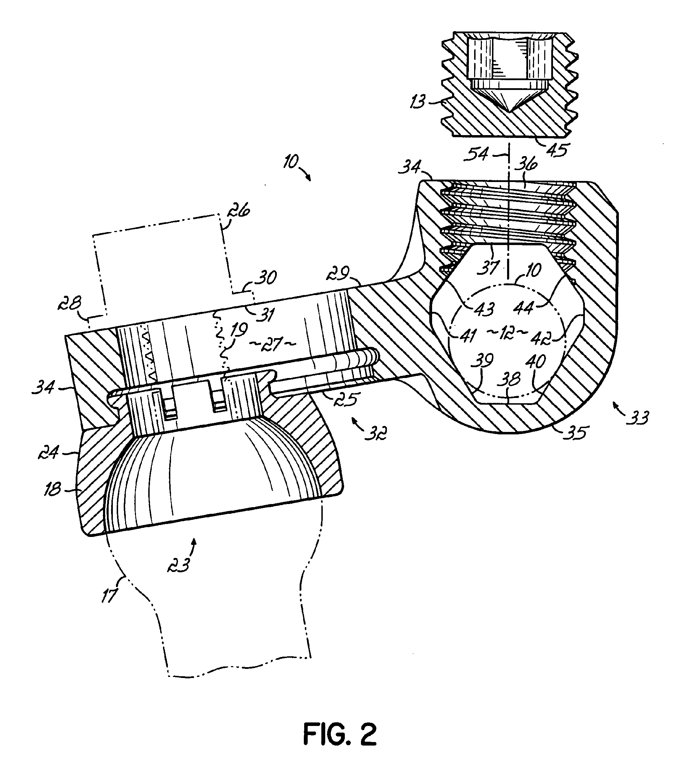

FIG. 1 shows one embodiment of this system. A vertebral anchor such as a polyaxial pedicle screw 14 is shown coupled to a connector 11 that in turn is adapted to receive a pre-shaped circular cross section support rod 10 (FIG. 2). The polyaxial screw 14 of one embodiment includes a threaded portion 15 in the form of a threaded shaft adapted for insertion into a human spinal column. A tip 16 of the screw 14 is adapted to be driven into a spinal column element. A spherically-shaped head 17 of the screw 14 mates with a polyaxial body 23 having a concavely shaped socket portion 18, and a threaded second portion 19 connecting the screw 14 to the connector 11.

The threaded portion 15 of the screw 14, as depicted in FIG. 1, has a varying cross sectional diameter along its longitudinal axis, whereby the diameter constantly decreases such that the threaded portion 15 is the widest in a first region 20 proximate to the point where the screw 14 is coupled to the polyaxial body and the narrowest in a second region 21 proximate the tip 16 of the screw 14. A person of ordinary skill in the art will appreciate the fact that any bone coupling anchor such as a hook, a clip, a bolt or a screw of a type different from the one in this embodiment may be employed.

The threaded portion 15 of the screw 14 depicted in FIG. 1 has a spherically-shaped head 17 adapted for coupling into a spherically or concavely-shaped receiving first end 18 of the polyaxial body 23. The outside surface 24 of the concavely-shaped receiving first end 18 of the body 23 mates with a first surface 25 of a flange portion 32 of the connector 11. This polyaxial body 23 has a threaded portion 19, adapted to be threadably coupled to an internally threaded lock nut 26 or similarly threaded member. In the construct of the embodiment of FIG. 1, the threaded portion 19 passes through an opening 27 in the connector 11 to receive a washer 28, which sits between a second surface 29 of the flange portion 32 of the connector and a lock nut 26. The internally-threaded lock nut 26 depicted in FIG. 1 threadably engages the threaded portion 19 of polyaxial body 23 of the screw 14 such that, when tightened, it exerts a force against a first surface 30 of a washer, a second surface 31 of which, being parallel to the first surface 30 of the washer 28 and to the first and second surfaces 25, 29 of the connector 11, contacts the second surface 29 of the flange portion 32 of the connector 11 and exerts a force against such second surface 29 of the connector 11, thereby frictionally securing the polyaxial body 23 against the connector 11.

The embodiment of FIG. 1, furthermore, shows a polyaxial screw 14, with a range of motion 31 of the threaded portion 15 of the screw 14 consistent with the angular motion permitted by the concavely shaped socket portion 18 of the screw 14. When inserted into the spinal column element, the angular orientation and depth of engagement of the threaded portion 15 of the screw 14 will be fixed by its unique position within the column. Similarly, the connector 11 will be fixed against motion relative to the screw 14 by the frictional force securing the connector 11 to the polyaxial body 23. Even though the described embodiment includes a polyaxial screw 14, a person of ordinary skill in the art may choose to replace this type of anchor with any other type of device suitable to be coupled to a spinal column or other bone.

The components of the connector assembly 11 could be made out of a metal, thermoplastics or any other surgical-grade materials. A metal connector 11, such as the one of the embodiment of FIG. 1, could be conceivably made by a casting process or other similar metal-forming processes. An alternate similar single-piece connector made out of a thermoplastic can be formed by any of the available plastic-molding processes known to a person of ordinary skill in the art. The connector 11 of the embodiment shown in FIG. 1 includes a flange portion 32 and a rod-coupling portion 33. The flange portion 32 of the connector 11 has first and second parallel-plane surfaces 25, 29 through which an oblong-shaped screw-receiving opening 27 extends with an axis traverse to both first and second parallel-plane surfaces 25, 29. A third side 34 adjoins both the first and second parallel plane surfaces 25, 29. The oblong opening 27 is shaped such that the lateral position of the polyaxial body 23 can be adjusted with respect to the connector 11 itself and ultimately with respect to the support rod 10 that the screw 14 and connector 11 are intended to secure.

A major benefit of this lateral adjustment feature lies in the ability of the surgeon implanting a spinal fixation system to make adjustments of the relative positions of the components even after the screws 14 have been inserted into the spinal column. By having this adjustment feature, therefore, the surgeon minimizes the probability and frequency of instances where an anchor 14 has to be reinserted into a slightly different spot, thereby preventing unnecessary drilling of the spine and consequential destruction of osseous matter. Although the embodiment depicted contains an oblong shaped opening 27, a person of ordinary skill in the art will realize that other options are known and available to provide the lateral adjustment that such opening 27 provides.

The rod-coupling portion 33 of the connector 11 of the embodiment of FIG. 1 includes a first substantially planar surface 34 and a second non-planar opposite surface 35. A pass-through aperture 12, adapted to receive the support rod 10, runs with an axis substantially parallel to both first and second surfaces 34, 35 of the rod-coupling portion 33 of the connector 11. A fastener or set screw receiving aperture 36 runs between the first surface 34 of the rod-coupling portion 33 of the connector 11 and an interior wall 37 of the rod pass-through aperture 12, and is substantially perpendicular to the axis of the rod-receiving aperture 12. The set screw receiving aperture 36 of this embodiment is internally threaded and adapted to receive a fastener such as an externally threaded set screw 13 with a length at least greater than the distance between the rod pass-through aperture wall 37 and the first surface 34 of the rod-coupling portion 33 of the connector 11. Although the embodiment shown has a set screw 13 running through the set screw receiving aperture 36, a person of ordinary skill in the art may conceivably replace the set screw 13 with any other surgical-grade fastener to achieve the same results.

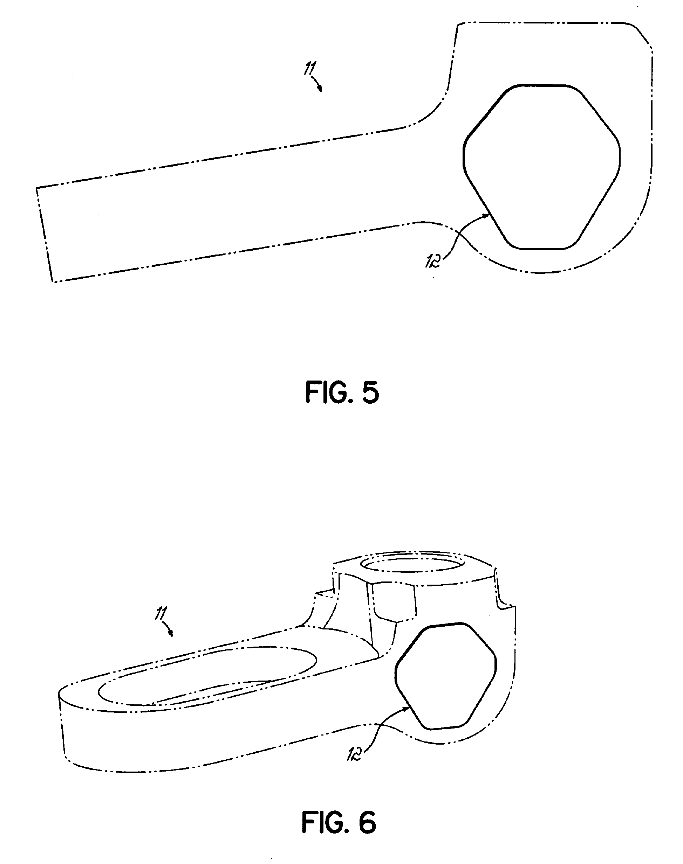

The cross-sectional shape of the rod pass-through aperture 12 of the embodiment can be better appreciated in FIGS. 2-6. The cross-sectional shape is polygonal and shown in this embodiment to be octagonal. Although the opening 12 is shown as having planar sides 37-44, a person of ordinary skill in the art may choose to make one or more of the aperture interior surfaces 37-44 non-planar, another shape and/or combine selected surfaces. An arcuate or convex wall, for example, could be substituted for one or more of the planar surfaces 37-44 forming the rod-receiving aperture 12 of this embodiment. This octagonal shape of the rod-receiving aperture 12 of this embodiment generally comprises a first pair of parallel planar walls 37, 38. Wall 37 is defined by the plane of entry 45 of the set screw 13 into the set screw receiving aperture 36 and wall 38 is diametrically opposed thereto. A second pair of parallel planar walls 41, 42 is perpendicular to the first pair 37, 38; a pair of planar walls 43, 44 adjacent to the plane of entry 45 of the set screw 13. A fourth pair of planar walls 39, 40 is made up of first and second lateral lines of contact each forming an acute angle with the axis of rotation 54 of the set screw 13.

The octagonal cross-sectional shape of the rod-receiving aperture 12 of this embodiment allows at least 3 spaced cross-sectional surfaces of contact between the connector 11 and the support rod 10 passing through the rod-receiving aperture 12, regardless of the diameter of the cylindrical support rod 10 used in the system. The surfaces of contact as shown in FIG. 2 are spaced, non-contiguous, separate and/or distinct from one another. As used herein, the term "surfaces of contact" means that the contact surfaces are spaced from one another. Otherwise, surfaces of contact which are not spaced from each other would actually be only a single contact surface. Nevertheless, such surfaces may be discrete points, lines, arcs, regions or other contact locations. Even though an octagonal shape rod-receiving aperture is described, other shaped rod-receiving apertures can be substituted.

Three inter-spaced surfaces of contact advantageously secure the connector 11 to the support rod 10. This particular octagonal embodiment, for example, will allow 3 spaced surfaces of contact for rods with a range of diameter between approximately 3mm. and approximately 6.35mm. The first surface of contact 46 is at a juncture between the rod 10 and the set screw 13. The second and third surfaces of contact 47, 48 are provided by the interface between the rod 10 and two non-contiguous planar surfaces 39, 40 substantially diametrically opposed to the plane of entry 45 of the set screw 13, also referred to as the first and second lines of lateral contact 39, 40. By allowing this inter-spaced 3-point contact 46-48, the rod 10 can be securely held by the set screw 13 and the two connector surfaces 39, 40 in the rod-receiving aperture 12, thus preventing any rotational or translational movement of the rod 10 with respect to the connector 11, thereby also preventing any rotational or translational movement of the rod 10 with respect to the screws 14 inserted in the spine.

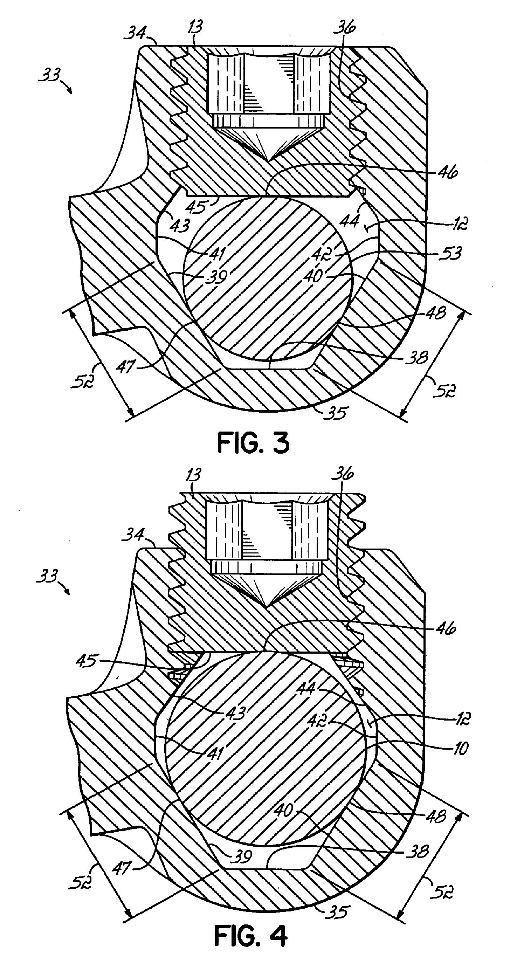

As will be appreciated, the rod-receiving aperture 12 of this embodiment can securely hold rods 10, 53 of different diameter. This flexibility is provided by the angular orientation of the first and second surfaces of lateral contact 39, 40 in the aperture 12 with respect to the axis of rotation 54 of the set screw 13, each of which allows a range of contact surfaces with the rod consistent with the length 52 of each of these walls 39, 40. A smaller rod 53, for example, will, as depicted in FIG. 3, contact the lateral surfaces 39, 40 at surfaces farther from the set screw 13, while a larger rod 10 will, as shown in FIG. 4, contact the lateral surfaces 39, 40 at surfaces relatively closer to the set screw 13. This flexibility is further facilitated by the range of longitudinal travel of the set screw along its axis of rotation 54, which permits the longitudinal position of the first surface of contact 46 to be adjusted to reach the surface of rods 10, 53 of different dimensions.

Moreover, while the rod 10 is shown and described herein as having a circular cross-sectional configuration with a generally smooth outer surface, other rod shapes and surfaces can be utilized. The rod surface may be roughened, knurled or otherwise configured to enhance gripping contact with the connector 11.

From the above disclosure of the general principles of this invention and the preceding detailed description of at least one embodiment, those skilled in the art will readily comprehend the various modifications to which this invention is susceptible.

## Claims

1. A spinal fixation system comprising:
at least two anchors adapted for coupling to a spinal column;
a support rod adapted to fix a relative position of vertebrae;
at least two connector assemblies each for connecting one of said anchors to said support rod; and
an aperture for said support rod/s in at least one of said connector assemblies, wherein an interface between the connector assembly and the rod passing through the aperture includes 3 surfaces of contact there between.

2. The system of claim 1, wherein the surfaces of contact are spaced from one another.

3. The system of either claim 1 or claim 2, wherein each connector assembly includes a fastener.

4. The system of claim 3, wherein said fastener is a set screw threadably received in said connector assembly and adapted to secure said rod in said aperture.

5. The system of either claim 3 or claim 4, wherein one of said surfaces of contact are between said fastener and the rod.

6. The system of any preceding claim, wherein at least one of said surfaces of contact is between the rod and one of a planar surface and an arcuate surface forming part of said aperture.

7. The system of any preceding claim, wherein each said anchor is one of a polyaxial screw, a uniaxial screw and a hook.

8. The system of any preceding claim, wherein said connector assembly allows for the distance between said anchor and a longitudinal axis of the rod to be adjustable.

9. The system of any preceding claim, wherein said connector assembly aperture is adapted to receive rods with a range of diameters from approximately 3mm to approximately 6.35mm.

10. The system of any preceding claim, wherein said connector assembly aperture is polygonal in shape.

11. The system of claim 10, wherein said polygonal aperture is defined in part by first, second and third serially connected planar surfaces and two of said surfaces of contact are provided by said first and third planar surfaces.

12. The spinal fixation system of claim 1 comprising an octagonal-shape aperture in each of said connector assemblies and a set screw in each of said connector assemblies for securing the rod against the walls of said aperture in the connector assembly, wherein a first of said surfaces of contact is between the rod and the set screw, and a second of said surfaces of contact is between the rod and a planar surface forming part of the aperture and spaced from said set screw.

13. A connector assembly for use in a spinal fixation system partially having a plurality of anchors coupled to a spine and a rod extending along the spine, the assembly comprising:
a connector body adapted to connect one of the anchors to the rod and having a portion adapted to be coupled to one of the anchors; and
an aperture in the connector body adapted to receive the rod, wherein an interface between the connector assembly and the rod includes 3 surfaces of contact there between.

14. The assembly of claim 13, wherein the surfaces of contact are spaced from one another.

15. The assembly of either claim 13 or claim 14, wherein the connector assembly includes a fastener.

16. The assembly of claim 15, wherein said fastener is a set screw threadably received in said connector assembly and adapted to secure said rod in said aperture.

17. The assembly of either claim 15 or claim 16, wherein one of said surfaces of contact is between said fastener and the rod.

18. The assembly of any one of claims 13 to 17, wherein at least one of said surfaces of contact is between the rod and one of a planar surface and an arcuate surface forming part of said aperture.

19. The assembly of any one of claims 13 to 18, wherein said anchor is one of a polyaxial screw, a uniaxial screw and a hook.

20. The assembly of any one of claims 13 to 19, wherein said connector assembly allows for the distance between said anchor and a longitudinal axis of the rod to be adjustable.

21. The assembly of any one of claims 13 to 20, wherein said connector assembly aperture is adapted to receive rods with a range of diameters from approximately 3mm to approximately 6.35mm.

22. The assembly of any one of claims 13 to 21, wherein the connector assembly aperture is polygonal in shape.

23. The assembly of claim 22, wherein said polygonal aperture is defined in part by first, second and third serially connected planar surfaces and two of said surfaces of contact are provided by said first and third planar surfaces.

24. The connector assembly of claim 13 comprising an octagonal-shape aperture adapted to receive the rod and a fastener for securing the rod against the walls of said aperture in the connector assembly, where a first of said surfaces of contact is between the rod and the set screw, and a second of said surfaces of contact is between the rod and a planar surface forming part of the aperture and spaced from said fastener.
